# EUROPEAN PATENT APPLICATION

(11) **EP 1 749 837 A1**
(43) Date of publication of application: **07.02.2007**
(21) Application number: 05076807.6
(22) Date of filing: 02.08.2005
(51) Int. Cl.: C07K 14/705, A61K 38/00

(54) **Methods and means for controlling the endocytosis and subsequent degradation of a cell surface protein**

(71) Applicant: Recharge B.V., 1391 CE Abcoude (NL)
(72) Inventor: Strous, Gerardus Jacobus Antonius Maria, 4175 AP Haaften (NL); van Kerkhof, Petrus Johannes Maria, 6658 DB Beneden Leeuwen (NL)
(74) Representative: Winckels, Johannes Hubertus F.

(57) **Abstract**

The invention relates to the field of regulating metabolic processes, for example to regulate the presence and/or activity of a receptor at the surface of a cell.

The invention provides a method for controlling the presence of a receptor at the surface of a cell comprising interfering with the interaction of an F-box protein with a ubiquitin/proteasome binding site of said receptor, wherein said ubiquitin/proteasome binding site comprises the amino acid sequence motif xDSWxEFIxxD or a sequence essentially corresponding thereto.

The invention further provides a method for controlling the presence of a growth hormone receptor (GHR) at the surface of a cell comprising interfering with the interaction of an F-box protein with a ubiquitin/proteasome binding site of said GHR.

## Description

The invention relates to the field of regulating metabolic processes, for example to regulate the presence and/or activity of a receptor at the surface of a cell.

A receptor (protein) on the surface of a cell has a binding site with, typically, a high affinity for a particular signalling substance (a hormone, pheromone, neurotransmitter, etc.). The specific signalling substance is often referred to as the ligand, a substance that binds to or fits in a site, the ligand binding site. When the signalling substance binds to a receptor, a receptor-ligand complex initiates a sequence or cascade of reactions that changes the function of the cell. A cell surface receptor polypeptide typically comprises an extra-cellular part which comprises a binding site where the ligand can interact, a transmembrane part, that locates a receptor in the cell membrane, and an intracellular part that plays a role in transducing a signal further into the cell once a ligand has bound. A receptor polypeptide can span the cell membrane several times resulting in multiple extra- and intracellular domains.

The response of a cell or tissue to for example specific hormones is dictated by the particular receptors it possesses and by the intra- or intercellular reactions initiated by the binding of any one hormone to its receptor. One cell may have two or more types of receptors or various cell types may have different sets of receptors for the same ligand, each of which induces a different response. Or the same receptor may occur on various cell types, and binding of the same ligand may trigger a different response in each type of cells. Clearly, different cells respond in a variety of ways to the same ligand, depending on a receptor or its interaction with the cell.

A wide variety of receptors specific for a wealth of ligands exist. Examples can be found among ion-channels, such as Ca⁺-channels, or Cl⁻ channels or Na⁺ channels, glucose transporters; among immunoglobulin receptors, such as IgE receptors; among cytokine receptors; among multi-drug transporters, and so on. Receptors, as defined herein, relate to signal transducing molecules in the broadest sense. These transducing molecules include ion-pump like proteins, for example the above-mentioned ion channels that transport the ligand (here the ion) through the membrane, include receptors that bind to a ligand (eliciting a signal over the membrane) but that do not transport the ligand itself through the membrane, include transport proteins and include enzymes that act upon contact with a substrate. As an example, receptors having as ligand a hormone are herein discussed in more detail, however, physiological mechanisms regulating hormone receptor availability and signal transduction are also found among a large variety of receptors having another ligand. As a non-limiting example, the growth hormone receptor is, further below, described in even more detail.

Hormones reacting with cell surface receptors are of a varied nature. Typical examples are amino acid derivatives such as epinephrine or histamine, prostaglandines, various peptide hormones such as glucagon, insulin, gastrin, secretin, ACTH, LH, FSH, TSH, TRH, LHRH, vasopressin, IGF-I or II, EGF, somatotropin (growth hormone), prolactin, erytropoietin, EGF, and others.

These hormones all act by binding to their specific surface receptor, after which their specific signal is being transduced, directly or by an intracellular signalling substance (a second messenger), leading to the specific action that is required of the cell.

The amount of functional (hormone) receptor on the cell surface is not constant. A receptor level is modulated up (up regulation) or down (down-regulation), permitting the cell to respond to small changes in the hormone (ligand) level. The number of cell surface receptors is often down-regulated by endocytosis, whereby the sensitivity of the cell for the specific hormone (ligand) is reduced.

In general, when a ligand (hormone) binds to its receptor and results in a ligand-receptor complex, two phenomena occur. On the one hand, the signal transduction cascade is initiated while on the other hand the ligand-receptor complexes are brought in the cell by receptor-mediated endocytosis and the internalised ligand (hormone) is degraded. Internalisation and degradation most likely terminate the hormone signal.

Some receptors recycle to the cell surface by exocytosis, however, even if they do, often a substantial fraction will be in the internal membrane compartments at any one time. Fewer receptors will be on the cell surface, available to bind extra-cellular hormone. Other receptors get degraded by proteolytic cleavage processes in the cell and thus do not or only insignificantly recycle to the cell surface, again reducing the number of available receptors on the cell surface.

Another way by which receptor availability on the cell surface is down-regulated is by removal, for example by specific proteolysis, of the extra-cellular part that comprises parts of the binding site of a receptor. Such removal is in essence a physiological mechanism that serves to refresh the available receptors and replace them with new ones, however, it again is a factor in reducing receptor availability.

As a consequence of fewer functional receptors being available on the cell surface, the hormone concentration necessary to induce the physiological response is higher and the sensitivity of the cell to the hormone is reduced. The susceptibility of a cell or tissue to the action of a hormone is thus among others dependent on the number of functional receptors present at any given time on the surface of a cell. Even when ligands are circulating at a high concentration, these cannot result in sufficient activation when not enough receptors are present.

Many hormonal related or other diseases would benefit from an up regulation of hormone or ligand activity. In hormonal dysfunctioning, one often attempts to achieve such up regulation simply by treating a patient with exogenous hormones, however, as explained above, such a treatment may not be effective due to the fact that the number of available surface receptors for that hormone are too low. This is often aggravated by the fact that higher hormone concentrations enhance, by feed-back mechanisms, the further down-regulation of the specific receptor. Exogenous hormone therapy may then even be counterproductive, the patient becomes less susceptible to the hormone in question.

As already described above, the growth hormone receptor is described in more detail to exemplify the present invention.

Cells of the human body contain growth hormone receptors (GHR). Upon growth hormone (GH) binding these receptors initiate signal transduction that results in the expression of genes that are involved in anabolic processes: protein synthesis, lipid degradation, etc. At the cell surface the expression of GHR is controlled by two major factors: (i) endocytosis and (ii) proteolytic cleavage of the extra cellular domain. In the first process the clathrin mediated endocytosis of the GHR depends on the activity of the ubiquitin system, and the second factor is the activity of TACE, tumour necrosis factor-α converting enzyme, also referred to as Adam17. Together, they largely determine the residence time of the GHR at the cell surface. Inhibition of both activities prolongs the half-life of the GHR from less than 1 hour to several hours. Inhibition of the ubiquitination-dependent endocytosis results in a two- to three-fold increase of GHRs at the cell surface (van Kerkhof et al., 2003).

A specific motif in the GHR cytoplasmic tail regulates receptor endocytosis via the ubiquitin-conjugation system. This motif, the Ubiquitin-dependent Endocytosis motif (UbE-motif), consists of the amino acid sequence **DDSWVEFIELD** (Govers et al., 1999). Mutational analysis of this motif has shown that the bolded and underlined amino acid residues are important for endocytosis and consequent degradation of the GHR in lysosomes. If these amino acid residues are mutated to alanine, endocytosis of the GHR dramatically drops, rendering the cells more GH sensitive. This finding was described in our patent application WO 99/46298.

The ubiquitin-proteasome degradation pathway provides the major pathway for non-lysosomal degradation, (reviewed in: (Hershko et al., 2000). It is involved in the degradation of cytoplasmic proteins and proteins, which do not pass the quality control of the ER, and plays a role in regulating a variety of cellular functions (Plemper and Wolf, 1999). Covalent attachment of the 76-amino acid peptide ubiquitin to substrate proteins is a highly conserved process that occurs via the sequential action of three enzymes: the ubiquitin-activating enzyme E1, an ubiquitin conjugase E2, and an ubiquitin ligase E3.

E3s are either single proteins, or multiprotein complexes. Two major classes exist: HECT E3s form a thioester bond with ubiquitin within the HECT domain during the transfer of ubiquitin from the E2 to the substrate, RING domain E3s have no intrinsic catalytic activity, but they coordinate the ubiquitin transfer by binding E2, serving as a scaffold. RING E3s consist of two subclasses: single peptide E3s (e.g. c-Cbl, Mdm2, Traf), and RING E3 complexes in which the substrate-recognition site and RING domain are in different proteins (e.g. SCF E3s). In SCF (Skp1/Cullin/F-box) complexes the F-box protein plays the role of recruiting on one site the cognate adapter protein and the rest of the E3 complex via its F-box motif of about 40 amino acids, on the other site the substrate.

Beta-transducing repeat-containing proteins (β-TrCP) serve as the substrate recognition subunits for the SCF^{TrCP} ligases. Two isoforms exist: β-TrCP1 is mainly present in the nucleus, while β-TrCP2 resides in the cytosol. These ligases play a role in cell division and various signal transduction pathways essential for many aspects of tumorigenesis. Their substrate binding motifs consist of seven propeller-shaped WD40 domains that specifically bind short motifs in its central cavity. Examples of substrates are β-catenin, NFκB, and inhibitor of NFκB. These motifs generally are 6 amino acid residue-long with a common structure of DSG(X)₂₊ₙS. To be recognized by the SCF E3 both serine residues need to phosphorylated; in some instances, recognition may also occur if the phosphorylated serines are replaced by an acidic amino acid residue. Recognition by SCF E3s results in poly-ubiquitination and degradation by the proteasome. In some cases the substrate is only partially degraded resulting in active (transcription) factors like NFκB.

Recently, it became evident that for a restricted number of plasma membrane proteins, ubiquitination triggers internalisation and vacuolar/lysosomal rather than proteasomal degradation, (reviewed in: (Strous and Govers, 1999). This pathway is best understood in yeast, where a number of plasma membrane proteins is endocytosed in a ubiquitin-dependent manner (Galan and Haguenauer-Tsapis, 1997; Hicke and Dunn, 2003; Terrell et al., 1998). Several studies showed that mono-ubiquitination of proteins is sufficient to stimulate their endocytosis (Galan and Haguenauer-Tsapis, 1997; Roth and Davis, 2000; Terrell et al., 1998). In mammalian cells, the GHR, the β-adrenergic receptor, and the epithelial sodium channel, ENaC, are cell surface proteins known to be endocytosed ubiquitin system-dependent (Shenoy et al., 2001; Staub et al., 1997; Strous and Gent, 2002). Ubiquitination of β-arrcstin by the E3 Mdm2 is required for endocytosis of the β2-adrenergic receptor, suggesting that the ubiquitination of an adaptor protein triggers endocytosis (Shenoy et al., 2001).

Recently, it became clear that the DSG(X)₂₊ₙS motif in some cytokine receptors serves as a degradation signal via the SCF(β-TrCP) ligases. Both for the interferon-α and for the prolactin receptor, it was shown that this E3 is involved in their degradation. An important finding in these studies was that receptor degradation mainly depends on ligand binding (interferon-α, prolactin, respectively); in addition, phosphorylation at both serine residues was required. This renders yet unidentified kinases the controlling factors for interferon/prolactin receptor degradation (Kumar et al., 2003; Kumar et al., 2004; Li et al., 2004).

β-TrCP is a factor in the degradation of prolactin and interferon receptors (Kumar et al., 2003; Kumar et al., 2004; Li et al., 2004). Fuchs et al. identified β-TrCP to act upon and interact with these receptors via their DSGΦXS destruction motifs. They also show that SCF(β-TrCP2) E3 ubiquitinates both the interferon-α and prolactin receptors, dependent on an intact DSGΦXS motif. The assumption is that upon ligand binding (interferon-α and prolactin) this motif is phosphorylated by a yet unidentified kinase after which the SCF(βTrCP2) E3 ubiquitinates certain lysines in the tail of the receptors.

Although the growth hormone receptor (GHR) comprises a DSGΦXS destruction motif, it is now surprisingly disclosed that a completely different motif plays a role in the ubiquitin-mediated proteolysis of a GHR. Moreover, it is also shown that β-TrCP is involved in the endocytosis of the GHR.

This finding let us to develop, amongst others, pharmacological tools or medication that can up- or down-regulate the presence of signal-transduction of ligand-specific proteins such as transport proteins, enzymes, cytosolic receptors or receptors on the surface of cells, for example to make a patient better responding to hormonal therapy or to provide the cells of said patient with a higher sensitivity to an endogenous hormone or other ligand.

In a first embodiment the invention provides a method for controlling the presence of a receptor at the surface of a cell comprising interfering with the interaction of an F-box protein with a ubiquitin/proteasome binding site of said receptor, wherein said ubiquitin/proteasome binding site comprises the amino acid sequence motif xDSWxEFIxxD or a sequence essentially corresponding thereto (such as DxSWxEFIxxD or DDSWxEFIxxD). It is now disclosed for the first time that an F-box protein is capable of interacting with (the intracellular part of) a receptor not only via a so-called DSGΦXS destruction motif, but also via the UbE motif or a sequence essentially corresponding thereto. This was highly unexpected, because the prolactine receptor which comprises a UbE motif as well as a DSGΦXS destruction motif is reported to be controlled via the DSGΦXS destruction motif. The GHR also comprises a UbE motif as well as a DSGΦXS destruction motif, but its presence at the surface of the cell is (as shown herein) (at least partly) controlled by an interaction between an F-box protein and the UbE-motif. Although we now have shown that the interaction of an F-box protein and the UbE motif plays a role in the amount of receptor present at the cell surface it is not to be excluded that any other sequence parts of said receptor may also play a role in the proteolysis of a receptor in general and of GHR in particular. For example, sequences present between the UbE motif of GHR and the transmembrane domain of GHR might play a(n) (additional) role. However, we have herein shown that some mutations of the UbE motif lead already to background signals.

Depending on the specific aim of a method according to the invention, the controlling step is to be understood as to result in either a decrease of the amount of receptor molecules present at the surface of a cell or to result in an increase of the amount of receptor molecules present at the surface of a cell or to result in a more or less constant/stable amount of receptor molecules.

Whether or not a receptor is present at the surface of a cell can be determined in different ways. The presence of a receptor at the surface of a cell is for exampled determined with help of antibodies (specifically) directed to said receptor or by binding studies using for example fluorescently or radioactively labelled GH.

Preferably, before a method of the present invention is applied, the amount of receptors at the surface of a cell is determined. This process is repeated after the interfering step. The amount of receptors before and after the interfering step is then compared and it is determined whether the desired controlling (up- or down regulation) has succeeded.

Whether the controlling step results in an increased/decreased/more or less constant amount (or level) of receptors at the surface of a cell is accomplished by interfering with the interaction of an F-box protein with a ubiquitin/proteasome binding site of said receptor. To decrease the amount of receptors present at the surface of a cell, said interaction is at least partly increased.

In a preferred embodiment, the amount of receptors present at the cell surface is increased. This is accomplished by at least in part decreasing the interaction between an F-box protein with a ubiquitin/proteasome binding site (preferably the UbE motif) of said receptor. "At least in part" is defined herein such that the interaction between an F-box protein and a ubiquitin/proteasome binding site is disturbed to such an extent that the presence of a receptor at the surface is (preferably significantly) increased. More preferably, said interaction is completely inhibited and hence the presence of a receptor at the surface of a cell is (preferably significantly) increased which renders the cell more sensitive to the corresponding ligand (GH in case in case of the GHR). The invention therefore provides a method for increasing the presence of a receptor at the surface of a cell comprising providing to said cell an inhibitor that at least in part is capable of inhibiting the interaction of an F-box protein with a ubiquitin/proteasome binding site of said receptor, wherein said binding site comprises the amino acid sequence motif xDSWxEFIxxD or a sequence essentially corresponding thereto.

Preferably, the inhibition of the interaction between said F-box protein and said ubiquitin/proteasome binding site (preferably the UbE motif) is such that only the amount of one particular receptor (preferably GHR) is changed without changing the amount of other receptor molecules or without interfering with the action of other molecules such as catenin, NFκB and the inhibitor of NFκB, the inhibitor of APC Emi1, the Cdc2 inhibitory kinase Weel, the transcription repressor Per-1, the transcription factor ATF4, and Cdc25B Phosphatase active at G2/M transition, i.e said inhibitor preferably specifically interferes with the binding of said F-box protein and UbE binding site of the to be modified (for example increased) receptor.

Preferably, said ubiquitin/proteasome binding site comprises the amino acid sequence motif xDSWxEFIxxD or a sequence essentially corresponding thereto which sequence is found in the intra-cellular polypeptide part of the receptor.

The herein used nomenclature for amino acid residues corresponds with the internationally accepted nomenclature and hence D is the amino acid aspartic acid, E is the amino acid glutamic acid, F is the amino acid phenylalanine, I is the amino acid isoleucine, S is the amino acid serine, W is the amino acid tryptophan and X is any other amino acid.

Essentially corresponding means herein that the amino acid motif relates to a variety of specific amino acid sequences. For example, the amino acid E (glutamic acid) in the motif can be replaced by the like amino acid D (aspartic acid), F (phenylalanine) can be replaced by Y (tyrosine), I (isoleucine) by L (leucine), V (valine) or F (phenylalanine), S (serine) by T (threonine), or D by E. A further detailed example is the amino acid sequence DxSWxEFIxxD or DDSWxEFIxxD or DDSWVEFIELD or DDSWVEFIELDI (the last two examples located, for example, at a distance of about 50 amino acid residues from the plasma membrane in the intracellular part of the growth hormone receptor).

Previously we have shown that endocytosis of the GHR depends both on a functional uibiquitination system and on an intact UbE motif (Govers et al., 1997; Strous et al., 1996; van Kerkhof et al., 2002). Using mutational analysis of the UbE motif by changing every single amino acid residue into alanine the contribution of each amino acid was determined. The experiment showed that mutation of some of the residues, indicated in bold (and underlined) in the UbE motif (D**DSW**V**EFI**EL**D**) almost completely inhibited endocytosis.

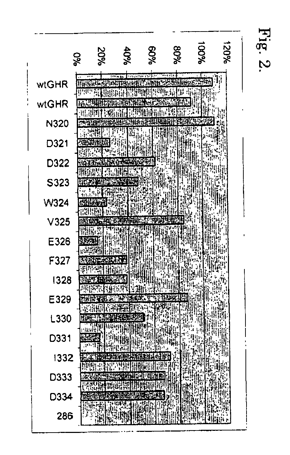

We now used a pull-down assay to determine whether β-TrCP binds to the GHR. Hek293 cells were transfected with 15 GHR constructs in with each of the amino acids of the UbE motif were changed into alanine (NDDSWVEFIELDIDD, 320-334). After 24h GHRs were isolated using biotinylated GH and incubated with equal amounts of β-TrCP, ³⁵S-labeled in an *in vitro* transcription-translation system. The bound radioactivity was analysed by electrophoresis on SDS gels and quantitated. As control DNA encoding wild-type GHR (Fig. 2, wtGHR) was used, and as a negative control DNA encoding GHR truncated at amino acid 286 (Fig. 2, 286), lacking the UbE motif. The results show that except for the N320A, every mutation resulted in inhibition of β-TrCP binding as compared to wtGHR. However, mutation of 7 amino acids inhibited the β-TrCP binding with more than 60% indicated as underlined DDSWVEFIELD, while the strongest effects were observed if either one of three acidic amino acid residues (D321, E326, D331) or S323, or W324 were mutated into alanine. The latter effects approximated the background signal measured for the GHR truncated at 286.

Therefore, in a preferred embodiment the invention provides a method for controlling the presence of a receptor at the surface of a cell comprising interfering with the interaction of an F-box protein with a ubiquitin/proteasome binding site (preferably the UbE motif) of said receptor or a method for increasing the presence of a receptor at the surface of a cell comprising providing to said cell an inhibitor that at least in part is capable of inhibiting the interaction of an F-box protein with a ubiquitin/proteasome binding site (preferably the UbE motif) of said receptor, wherein said ubiquitin/proteasome binding site comprises the amino acid sequence motif DxSWxEFIxxD or a sequence essentially corresponding thereto, such as the amino acid sequence motif DDSWxEFIxxD or the amino acid sequence motif DDSWVEFIELD.

We now disclose that the UbE motif in the GHR tail is specifically recognized by β-TrCP and hence in a preferred embodiment the mentioned F-box protein is a β-transducing repeat-containing protein (β-TrCP). Even more preferably the to be controlled receptor is a growth hormone receptor (GHR).

A method according to the invention can be performed *in vitro* as well as *in vivo.* The *in vitro* method is, for example, extremely useful for the testing of compounds that are possible inhibitors of the interaction between an F-box protein and a ubiquitin/proteasome binding site of a, to be controlled, receptor. The *in vivo* method is, for example, useful for example to make a patient better responding to hormonal therapy or to provide the cells of said patient with a higher sensitivity to an endogenous hormone or other ligand.

The inhibitor used in a method according to the invention can be a peptide or peptide analogue or mimeticum that is derived from, competes with or binds to any of the above-mentioned ubiquitin/proteasome binding site. We have determined that, for example, a peptide consisting of the amino acids 270-331 (61 amino acids) of GHR is capable of providing inhibition. The sequence of human 61 amino acid peptide is:
270-KQQRIKMLIL PPVPVPKIKG IDPDLLKEGK LEEVNTILAI
HDSYKPEFHS DDSWVEFIEL D-331 and the sequence of the rabbit 61 amino acid sequence is:
270-KQQRIKMLIL PPVPVPKIKG IDPDLLKEGK LEEVNTILAI QDSYKPEFYN DDSWVEFIEL D-331
A skilled person is very well capable of making smaller peptides or derivatives based on the sequence of this 61 amino acid peptide of GHR. Such peptides are also part of the invention. Such a smaller peptide comprises between 5-50 amino acids or 5-40 amino acids or 5-30 amino acids, preferably between 5-20 amino acids, more preferably between 5-10 amino acids and most preferably between 5-15 amino acids. Methods to test such peptides for their capability to inhibit the interaction between for example the GHR and β-TrCP are provided herein.

Such a peptide, peptide analogue or mimeticum is preferably provided with a means for transport across the cell membrane. A non-limiting example is the HIV-TAT domain. A peptide or peptide analogue or mimeticum that is derived from the ubiquitin/proteasome binding site (xDSWxEFIxxD or DxSWxEFIxxD or DDSWxEFIxxD or DDSWVEFIELD) is preferably designed such that it has 70 % homology with said ubiquitin/proteasome binding site, more preferably 80%, even more preferably 90% and most preferably 95% homology.

The invention thus also provides a peptide or peptide analogue or mimeticum derived from an amino acid sequence that corresponds to the amino acid motif xDSWxEFIx3cD or DxSWxEFIxxD or DDSWxEFIxxD or DDSWVEFIELD or to a motif essentially corresponding thereto (as defined herein). A peptide that competes, binds, or interacts in another way with such an amino acid sequence is herein also considered to be derived from an amino acid sequence corresponding to such a motif. Such a peptide is selected according to methods known by a person skilled in the art. It is to be expected that the conformation of a selected peptide is important for its reactivity. Appropriate conformational changes can be introduced in selected peptides by techniques known to the person skilled in the art, for example it is possible to introduce appropriate conformation by using disulfide bridges. Also other peptide sequences or compounds, mimicking the wanted conformation (mimetica) can be selected by a person skilled in the art. A suitable system to select a peptide is a system such as the PEPSCAN system, whereby interaction, such as competition or inhibition, is measured against sets of overlapping peptides chosen from the receptor's amino acid sequence. Peptide analogous, whereby specific amino acids are replaced by others, being either L-or D-amino acids, are tested similarly. Other methods include replacement scanning of selected peptide sequences, for example by replacing distinct amino acids by alanine, whereby crucial amino acids in the selected peptides are determined. Peptides can be made synthetically or via recombinant techniques.

In yet another embodiment, the inhibitor is a small (chemical) compound which is for example obtained from a small-compound library. Methods to identify such compounds will be discussed in more detail below.

In another embodiment, the inhibitor is derived from an antibody (or more preferably a part thereof) directed against the ubiquitin/proteasome binding site or directed to the part of β-TrCP that interacts with the UbE motif. It is clear to the skilled person, that preferably the antibody (or part thereof) is directed against the ubiquitin/proteasome binding site, to avoid any undesired side effects by interfering with the other functions of an F-box protein. A skilled person is very well aware of the way in which antibodies (or parts thereof) can be raised and hence no further details will be provided on this matter. Preferably, the raised antibodies are monoclonal antibodies. Antibodies or a part thereof can further be provided with a means that facilitate transport across the cell membrane (for example HIV TAT domain). A non-limiting example of a useful part of an antibody is the CDR3 region of the heavy chain which specifically interacts with at least part of the ubiquitin/proteasome binding site or which is capable of binding to a dimerised ubiquitin/proteasome binding site.

In yet another preferred embodiment the inhibitor is a compound that binds (more or less) irreversible to the UbE-motif (or a sequence essentially corresponding thereto). Such an inhibitor has overall basic characteristics and is capable of forming hydrophobic interactions with the amino acids F and/or W of the UbE motif. By (more or less) irreversible blocking the UbE-motif, the F-box protein is not able to interact with said UbE-motif and as a result the corresponding receptor molecule will not be removed from the cell surface; this results in a prolonged presence of said receptor at the surface of a cell.

In yet another preferred embodiment the inhibitor is a compound that is capable of cross-linking two receptor molecules approximately around the interaction site of the receptor with the F-box protein. Especially GHR is present at the cell surface in a dimerised form (in the presence as well as in the absence of GH). Such an inhibitor results in a conformational change in the receptor which (at least partly) blocks the interaction with the F-box protein. Again, this results in a prolonged presence of said receptor at the cell surface.

As a general comment on the inhibitor it is noted that preferably the action of the inhibitor does not or at least not substantially disrupt the capabilities of the receptor to, after ligand binding, initiate the signal transduction cascade.

Dependent on the specific type of inhibitor, the inhibitor either blocks the ubiquitin/proteasome binding site by interacting/binding with/to said site such that β-TrCP can no longer functionally interact with the ubiquitin/proteasome binding site or the inhibitor (specifically) interacts with the F-box protein (β-TrCP) such that the F-box protein can no longer functionally interact with the ubiquitin/proteasome binding site/UbE motif of the receptor. In the last situation, the inhibition is preferably specific, such that the interaction of the F-box protein with other binding sites are not or hardly not affected. In either case, the action of the inhibitor results in more receptors at the surface of a cell or for a prolonged presence of a certain receptor at the surface of a cell and thereby making the cell more responsiveness to the corresponding ligand (in case of GHR: growth hormone).

In a second embodiment, the invention provides a method for controlling the presence of a growth hormone receptor (GHR) at the surface of a cell comprising interfering with the interaction of an F-box protein with the ubiquitin/proteasome binding site (preferably UbE motif) of said GHR. It is disclosed herein, to our knowledge for the first time, that an F-box protein is involved in endocytosis of the GHR by interacting with such a binding site of said GHR. Based on this finding, the amount of GHR can now be controlled by interfering with this interaction.

Preferably, the amount of GHR at the cell surface is at least partly increased by at least partly blocking the interaction between an F-box protein and a ubiquitin/proteasome binding site of GHR with help of an inhibitor and hence the invention provides a method for increasing the presence of a GHR at the surface of a cell comprising providing to said cell an inhibitor that at least in part is capable of inhibiting the interaction of an F-box protein with the ubiquitin/proteasome binding site /UbE motif of said GHR. More preferably, said F-box protein is a β-transducing repeat-containing protein (β-TrCP). Even more preferably, the mentioned ubiquitin/proteasome binding site comprises the amino acid sequence motif xDSWxEFIxxD or the amino acid sequence DxSWxEFIxxD or the amino acid sequence DDSWxEFIxxD or a sequence essentially corresponding thereto (as outlined above). In an even more preferred embodiment, the invention provides a method for controlling the presence of a GHR at the surface of a cell comprising interfering with the interaction of an F-box protein with the ubiquitin/proteasome binding site /UbE motif of said GHR, wherein said ubiquitin/proteasome binding site comprises the amino acid sequence motif DDSWVEFIELD or a sequence essentially corresponding thereto.

As outlined above, such a method can be *performed in vitro* as well as *in vivo.*

In yet another embodiment the invention provides a method for selecting an inhibitor capable of inhibiting the interaction of an F-box protein with the ubiquitin/proteasome binding site/UbE motif, comprising the steps of
- contacting, in the absence or presence of a possible inhibitor, said F-box protein or a functional part and/or equivalent thereof with a first labelled compound comprising at least a proteinaceous part comprising the amino acid sequence xDSWxEFIxxD or a sequence essentially corresponding thereto and
- determining the amount of said first labelled compound bound to said F-box protein in the presence or absence of said inhibitor and comparing the obtained amounts
- selecting the inhibitor that results in a decreased amount of said bound first labelled compound.
   Such a method is very useful in large-scale and/or high throughput screenings and hence allows for the screenings of libraries of possible inhibitors, for example small (chemical) compound libraries or peptide libraries or libraries containing natural and organic substances/products.
   The first labelled compound can be in very different forms but at least comprises a proteinaceous part. The proteinaceous part preferably comprises the amino acid sequence xDSWxEFIxxD or a sequence essentially corresponding thereto. Examples of other useful sequences are the amino acid sequences DxSWxEFIxxD or DDSWxEFIxxD or DDSWVEFIELD. The amino acid sequence may optionally be somewhat extended at the N- and/or C-terminus by for example 2 or 3 extra amino acids. The first labelled compound that comprises at least a proteinaceous part comprising the amino acid sequence xDSWxEFIxxD may also be the complete (optionally dimerized) GHR or the intracellular part of GHR.
   The first labelled compound further comprises a label. Examples of suitable labels are fluorescent labeled such as Cy3, Cy5 or Alexa-based labels or radioactively labels.
   Preferably, said F-box protein or a functional part and/or equivalent thereof is β-TrCP or a functional part and/or equivalent thereof. A functional part and/or equivalent thereof is for example detected by using mutations and deletions of β-TrCP. Although not described in detail in the experimental part, we have shown that the interaction between the xDSWxEFIxxD amino acid sequence and β-TrCP occurs via the WD40 domain of β-TrCP. In a more preferred embodiment, the functional part of β-TrCP comprises the WD40-domain.
   In view of the first labelled compound comprising at least a proteinaceous part comprising the amino acid sequence xDSWxEFIxxD or a sequence essentially corresponding thereto and in view of the F-box protein or a functional part and/or equivalent thereof, it is clear that different combinations can be used in a method for selecting an inhibitor capable of inhibiting the interaction between an F-box protein and a ubiquitin/proteasome binding site/UbE-based motif. Non-limiting examples include (i) the use of a complete F-box protein in combination with a first labelled compound comprising only the amino acid sequence xDSWxEFIxxD (or a sequence essentially corresponding thereto), possible extended by a limited (2-3) amount of amino acids on the N- and/or C-terminus or (ii) the use of a complete F-box protein in combination with a first labelled compound comprising essentially the complete intracellular domain of for example GHR or comprising essentially the complete GHR molecule or (iii) the use of a functional part and/or equivalent of an F-box protein (for example WD40 of β-TrCP) in combination with a first labelled compound comprising only the amino acid sequence xDSWxEFIxxD (or a sequence essentially corresponding thereto), possible extended by a limited (2-3) amount of amino acids on the N-and/or C-terminus.
   By comparing the amount of signal resulting from an incubation between an F-box protein (or a functional part and/or equivalent thereof) and a first labelled compound in the absence of a possible inhibitor (or in the presence of a non-inhibiting compound) with the amount of signal obtained in the presence of a possible inhibitor it can be determined whether a possible inhibitor indeed has inhibitory capabilities or not. If the signal of the incubation of the first labelled compound (bound to said F-box protein or a functional part and/or equivalent thereof) in the absence of a possible inhibitor and in the presence of said inhibitor are approximately the same, said inhibitor will not be useful for further uses. However, if the signal obtained in the presence of a possible inhibitor is (much) lower compared to the signal obtained in the absence of said inhibitor, a possible useful inhibitor is identified.
   Preferably, a method for selecting an inhibitor as described above, further comprises immobilising said F-box protein or a functional part and/or equivalent thereof. This enables an easy separation of bound and unbound first labelled compound, for example by performing one or multiple washing steps. Suitable, non-limiting, matrices for immobilisation of said F-box protein are ELISA plates, membranes, arrays and other solid-phase-based media.
   Instead of using a first labelled compound comprising at least a proteinaceous part comprising the amino acid sequence xDSWxEFIxxD or a sequence essentially corresponding thereto it is clear that a method for selecting an inhibitor capable of inhibiting the interaction of an F-box protein with the ubiquitin/proteasome binding site/UbE motif is equally well performed with a labelled F-box protein or a functional part and/or equivalent thereof. Thus the invention also provides a method for selecting an inhibitor capable of inhibiting the interaction of an F-box protein with a ubiquitin/proteasome binding site, comprising the steps of
- contacting, in the absence or presence of a possible inhibitor, said ubiquitin/proteasome binding site (or preferably the UbE motif) comprising the amino acid sequence xDSWxEFIxxD or a sequence essentially corresponding thereto with a first labelled compound comprising said F-box protein or a functional part and/or equivalent thereof and
- determining the amount of said first labelled compound bound to said ubiquitin/proteasome binding site in the presence or absence of said inhibitor and comparing the obtained amounts
- selecting the inhibitor that results in a decreased amount of said bound first labelled compound.
   Useful labels for the F-box protein or a functional part and/or equivalent thereof are outlined above. In a preferred embodiment said F-box protein is β-TrCP and said functional part of said F-box protein comprises the WD40-domain.
   Again, different combinations of the first labelled compound and a ubiquitin/proteasome binding site can be used. Non-limiting examples are include (i) the use of a labelled, essentially complete, F-box protein as a first labelled compound in combination with a compound comprising the amino acid sequence xDSWxEFIxxD (or a sequence essentially corresponding thereto), possible extended by a limited (2-3) amount of amino acids on the N- and/or C-terminus or (ii) the use of a labelled, essentially complete, F-box protein as a first labelled compound in combination with a compound comprising essentially the complete intracellular domain of for example GHR or comprising essentially the complete GHR molecule or (iii) the use of a functional part and/or equivalent of an F-box protein (for example WD40 of β-TrCP) as a first labelled compound in combination with a compound comprising only the amino acid sequence xDSWxEFIxxD (or a sequence essentially corresponding thereto), possible extended by a limited (2-3) amount of amino acids on the N- and/or C-terminus.
   Preferably, such a method further comprises immobilising said (compound comprising a) ubiquitin/proteasome binding site.
   Preferably, the selected inhibitor does not interfere with other processes in which for example the β-TrCP molecule is also involved and hence the method for selecting an inhibitor capable of inhibiting the interaction of an F-box protein with a ubiquitin/proteasome binding step further comprises
- contacting, in the absence or presence of the selected inhibitor, said F-box protein or a functional part and/or equivalent thereof with a second labelled compound comprising at least a proteinaceous part comprising the amino acid sequence DSGIHS or a sequence essentially corresponding thereto,
- determining the amount of said second labelled compound bound to said F-box protein in the presence or absence of said inhibitor and comparing the obtained amounts,
- selecting the inhibitor that does not or hardly not change the amount of said second labelled compound bound to said F-box protein.

Examples of useful labels are the same as the labels described for the first labelled compound.

Selected inhibitors are optionally further tested in a cell-based assay or in an animal model for their effectiveness/usefulness.

The selected inhibitors can optionally be modified, for example by providing the inhibitor with a means to (more efficiently) cross the cell membrane.

The invention further provides an inhibitor obtainable by a method according to the invention. Such an inhibitor may be proteinaceous or chemical, synthetically produced or obtained via recombinant techniques. Such an inhibitor may further be provided with a means to increase the inhibitor's potential to cross the cell membrane. Such inhibitors are also useful in the design of mimeticum.

The invention further provides a pharmaceutical composition comprising an inhibitor according to the invention. Such a pharmaceutical composition is for example useful in treating patients with hormone (for example growth hormone) deficiencies.

The invention also provides use of an inhibitor according to the invention for the manufacture of a medicament for treating diseases related to the availability (presence) of a cell surface receptor. More in particular, the invention provides use of an inhibitor according to the invention for the manufacture of a medicament for the treatment of (diseases which involve) cachexia. Even more in particular, the invention provides use of an inhibitor according to the invention for the manufacture of a medicament for the treatment of diseases which involve cachexia, wherein said cachexia is a result of cancer, AIDS, renal insufficiency, burns or bed-ridden situations. Yet another example of use of a pharmaceutical composition provided by the invention is for the treatment of patients that are suffering from (muscle) wasting that results from increased (muscle) protein degradation that is often seen after or during disorders such as renal tubular defects, uraemia, diabetes, Cushing's syndrome, cachexias seen with cancer or with eating disorders, after serious burns, during sepsis or AIDS, after stress, during and after immobilisation, during neuromuscular disease, and other conditions that alter protein degradation in cells such as muscle cells. Experimental animal models are available to study these and other related disorders. One can for instance use rats to study fasting, metabolic acidosis, kidney failure, muscle denervation, diabetes, thermal injury, endotoxaemia, bacteraemia, tumour development, glucocorticoid or thyroid hormone treatment and hyperthyroidism. Studies of such experimental models have indicated that the ubiquitin/proteasome pathway is activated in muscle and causes the loss of muscle mass (wasting) in these disorders.

Yet another embodiment of the invention is a pharmaceutical composition comprising an inhibitor provided by the invention which composition is administered in conjunction with a hormone. In conjunction herein meaning that said composition is used or applied before, during or after hormonal treatment and up-regulates or modifies the activity of said hormone.

The invention also provides for optimalisation of physical and mental condition in healthy individuals to increase lean body mass and diminish body fat without the use of GH. A selected inhibitor that is derived from a naturally occurring product is used as a dietary supplement. Such a supplement preferably has a positive effect on the properties of muscles and/or reduces the amount of fat.

The invention further provides means and methods for the treatment of acromegaly. Acromegaly is a hormonal disorder that most commonly occurs in middle-aged men and women. The name "acromegaly" comes from the Greek words for "extremities" (acro) and "great" (megaly), because one of the most common symptoms of this condition is abnormal growth of the hands and feet. The symptoms of acromegaly can vary and they develop gradually over time; therefore, a diagnosis of this condition may be difficult. Early detection is a goal in the management of acromegaly because the pathologic effects of increased growth hormone (GH) production are progressive. Because we now know that β-TrCP interacts with ubiquitin/proteasome binding site of GHR, β-TrCP can now be modified to be more active, i.e. increase of the cellular concentration of β-TrCP (for example by increasing expression or by a decreased degradation).

The invention will be explained in more detail in the following description, which is not limiting the invention.

### Materials and methods

### Material

TrCP1 and TrCP2 cDNA in pcDNA1 expressing the flag-tagged proteins were generous gifts of Tomoki Chiba, Tokyo Metropolitan Institue of Medical Science. Full-length rabbit GHR cDNA in pcDNA3 was described (Strous et al., 1996). GST fusion proteins expressing GST-GHR(271-334), GST-GHR(271-334)F327A , and GST-GHR(271-318) were produced as described (Schantl et al., 2003). pcDNA3-GHR constructs used for alanine scanning of the UbE motif were constructed as described in Govers et al. (1999). Chemically synthesized siRNA duplexes to silence both β-TrCP1 and -2, GUGGAAUUUGUGGAACAUCtt, and GAUGUUCCACAAAUUCCACtt, and a control siRNA designed for GFP silencing were purchased from Ambion, Austin Texas. Antibody, recognizing the cytoplasmic domain of the GHR, was raised against amino acids 271-318 of the cytosolic tail of the GHR (van Kerkhof et al., 2000). Antibody (Mab5) directed against the extracellular domain of the GHR was from AGEN Inc. (Parsippany, NJ). Mouse monoclonal anti-FLAG (M2) were from Sigma. MG132 (carbobenzoxy-L-leucyl-L-leucyl-L-leucinal) was purchased from Calbiochem-Novabiochem, cycloheximide was purchased from ING Biomedicals Inc. Culture media, fetal calf serum (FCS), L-glutamine, and antibiotics for tissue culture were purchased from Gibco.

### Cell culture

Hek293 cells were grown in DMEM (Gibco) supplemented with 10% FCS, 100 units/ml Penicillin and 100 µg/ml streptomycin. Hek293 cells stable expressing the wtGHR (Hek-wtGHR) were grown as in the same medium supplemented 600 µg/ml Geneticin (G418; Gibco).All cells used were grown at 37°C with 5.0% CO₂ in 25 cm² or 75 cm² culture flasks. Once or twice a week the cells were washed with phosphate buffered saline (PBS), detached from the flask with Trypsin-EDTA (Gibco), diluted in fresh growth medium and split into new culture flasks. Growth medium was refreshed twice a week.

### Transfections

Hek293 cells were transfected using FuGene6 (Roch applied science). Fifty percent confluent cultures were transfected with 0.2 µg - 0.9 µg DNA in 12-well plates according to manufacturer's protocol. Transfected cells were used for Western blot experiments and ¹²⁵I-GH binding experiments 1-2 days after transfection.

To silence the expression of both β-TrCP1 and -2 Hek-wtGHR cells were transfected with small interfering RNA (siRNA) using Lipofectamin2000 according to the description of the manufacturer; 3 days after transfection the cells were used for Western blotting and ¹²⁵I-GH binding experiments. Control cells were transfected with siRNA-GFP. To control the effect of the gene expression silencing Hek293 cells were transiently transfected with a pcDNA3-TrCP construct expressing FLAG-tagged TrCP. Western-blotting using anti-Flag-tag antibody showed that less than 5% of TrCP present in cells treated with the control siRNA.

### ¹²⁵I-GH binding en internalization

¹²⁵I-Human GH was prepared using chloramine T (Strous et al 1996). For internalization experiments, cells, grown in 12-well plates, were washed with 1 ml MEM supplemented with 20 mM Hepes, pH 7.4 and 0.1% bovine serum albumin (BSA), and incubated at 37°C in a water bath for 1 hour. After washing the cells three times with ice-cold PBS-complete (PBS with 0,135g/l CaCl₂ and 0,1 g/l MgCl₂), 750 µl ¹²⁵I-GH (180 ng/ml in Mem/Hepes/0.1% BSA) was added to the cells. ¹²⁵I-GH was bound for 2 hours on ice. Unbound ¹²⁵I-GH was aspirated and the cells were washed three times with PBS-complete. Cells were incubated 0 to 10 minutes at 37 °C in MEM/Hepes/0,1% BSA to allow receptor internalization. Cells were washed three times with ice-cold PBS-complete and membrane associated ¹²⁵I-GH was removed by treating the cells twice with 750 µl acid wash (0.15 M NaCl, 50 mM glycine, 0.1% BSA, pH 2.5) for 5 minutes on ice. Acid wash is collected in counting tubes and radioactivity was measured using a LKB gamma counter. Acid wash treated cells were solubilized overnight in 1N NaOH. Internalized ¹²⁵I-GH was determined by measuring the radioactivity in the collected NaOH. Unspecific counts were determined by incubating the cells with ¹²⁵I-GH together with excess unlabeled GH (9 µg/ml). Internalization is expressed as a percentage of total specific radioactivity associated with the cells at 10 min at 37 °C.

### In vitro binding assay

Wild-type and mutant GHR proteins were expressed in Hek293, the cells were lysed in 50 mM Tris HCl (pH 7.5), 1 M NaCl, 50 mM NaF, and 0.5% Nonidet P-40 and the receptors were purified with biotinylated GH and streptavidin beads, stringently washed with lysis buffer. The beads were incubated with in vitro-translated and ³⁵S-labeled -TrCP2 for 60 min at 4°C. The beads were extensively washed with lysis buffer, and associated proteins were analyzed by sodium dodecyl sulfate-polyacrylamide gel electrophoresis (SDS-PAGE) and autoradiography. cDNA of β-TrCP2 constructs in pcDNA3.1/FLAG was used in an in-vitro transcription translation system supplemented with [³⁵S]methionine and TNT®-T7 coupled reticulocyte lysate ready reaction mix according to the instructions of the manufacturer (Promega). Binding to GHR1-286 was taken as back-ground.

### Results

### The involvement of β-TrCP in the endocytosis of the GHR

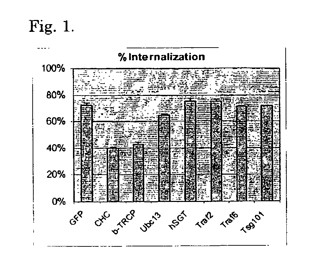

In a first experiment the role of β-TrCP in the endocytosis of the GHR was investigated. A Hek 293 cell line expressing the rabbit GHR was transfected with siRNA for 3 days, and uptake of ¹²⁵I-GH was measured for 10 min. As a negative control the expression of clathrin was silenced (CHC); in another control siRNA for green fluorescent protein was used (GFP). As seen in the Figure 1, SiRNA for β-TrCP inhibits GH uptake to the same extend as siRNA(CHC), approximately 50% as compared to non-relevant siRNA (GFP). Silencing of various other genes like the E2 Ubc13, the RING E3s Traf2 and Traf6, and the endosomal cargo selector complex ESCRT2 protein Tsg101 did not affect GH uptake.

We conclude that β-TrCP is involved in endocytosis of the GHR.

### Mutational analysis of the UbE motif

Previously we have shown that endocytosis of the GHR depends both on a functional uibiquitination system and on an intact UbE motif (Govers et al., 1997; Strous et al., 1996; van Kerkhof et al., 2002). Using mutational analysis of the UbE motif by changing every single amino acid residue into alanine the contribution of each amino acid was determined. The experiment showed that mutation of some of the residues, indicated in bold (and underlined) in the UbE motif (D**DSW**V**EFI**EL**D**) almost completely inhibited endocytosis. As shown in Figure 1, β-TrCP is acts as a regulator of GHR endocytosis. We used a pull-down assay to ask whether β-TrCP binds to the GHR in a specific fashion. Hek293 cells were transfected with 15 GHR constructs in with each of the amino acids of the UbE motif were changed into alanine (NDDSWVEFIELDIDD, 320-334). After 24h the GH-GHR complexes were isolated and incubated with equal amounts of β-TrCP, ³⁵S-labeled in an in vitro transcription-translation system. The bound radioactivity was analysed by electrophoresis on SDS gels and quantitated. As control DNA encoding wild-type GHR (Fig. 2, wtGHR) was used, and as a negative control DNA encoding GHR truncated at amino acid 286 (Fig. 2, 286), lacking the UbE motif. The results show that except for the N320A, every mutation resulted in inhibition of β-TrCP binding as compared to wtGHR. However, mutation of 7 aminoacids inhibited the β-TrCP binding with more than 60% indicated as underlined DDSWVEFIELD, while the strongest effects were observed if either one of three acidic amino acid residues (D321, E326, D331) or S323, or W324 were mutated into alanine. The latter effects approximated the background signal measured for the GHR truncated at 286. No binding was observed for the DSGRTS motif at postion 365, (not shwon). Further domain analysis using mutations and deletions of β-TrCP showed that the interaction between the UbE motif and β-TrCP occurs via its WD40 domain (not shown).

From comparison between the mutational analyses previously measured for GHR endocytosis and the results obtained with the same constructs for GHR-β-TrCP interaction, we conclude that β-TrCP is a prime regulator of GHR endocytosis via the UbE motif (DDSWVEFIELD).

Previously, we have shown that GHR endocytosis and subsequent degradation in the lysosomes depends on the activity of the ubiquitin system via the UbE motif. Here we identify β-TrCP as the factor that is required for GHR endocytosis by gene silencing, and that specifically binds to the GHR at the same motif as required for ubiquitination-dependent endocytosis. These data support the conclusion that this factor recruits the ubiquitination machinery. Whether the complete SCF(β-TrCP) complex including skp1, cul1, rbx1, and an E2 conjugase is present at the GHR tail remains to be determined.

The interaction between β-TrCP and the UbE motif is via the WD40 domain. Detailed structural information is available on the interaction between the WD40 domain of the SCF(β-TrCP) with β-catenin (Wu et al., 2003). The F box protein β-TrCP recognizes the doubly phosphorylated DpSGΦXpS destruction motif, present in β-catenin, and directs the SCF β-TrCP E3 to ubiquitinate it at a lysine residue 13 amino acids upstream of the destruction motif (the lysine residue is commonly present 9-14 position upstream). The β-catenin peptide binds the top face of the β-propeller, with the six residue destruction motif dipping into the central channel. The middle portion of the peptide has an extended conformation, and the two ends have turn-like conformations. All seven WD40 repeats of β-TrCP contribute contacts to β-catenin. Comparison with the UbE motif reveals little resemblance to the catenin destruction motif: the UbE motif is 11 amino acid long, it contains 3 acidic residues critical for binding (D321, E326, D331) instead of the two phosphorylated serine residues in β-catenin, and lacks a glycine residue, present in all phosphorylated DSGΦXS destruction motifs described until now ((Fuchs et al., 2004; Kanemori et al., 2005)). Another deviation is the lack of a lysine residue at 9-14 positions upstream, although there is a conserved lysine at 315 immediately after a tyrosine that is phosphorylated upon GH binding. However, mutation of this lysine residue into arginine does not affect the half life of the GHR, nor its ubiquitination-dependent endocytosis behaviour (Govcrs et al., 1999). Together, these results are in line with our previous observation that ubiquitination of the GHR is not required for endocytosis. Thus, binding of a presumed SCF(β-TrCP) E3 does not target the GHR for ubiquitination but is probably directed towards another factor in the GHR-ubiquitination complex (Govers et al., 1999). Before 3-D will reveal the precise interaction between the β-TrCP- WD40 domain and the UbE motif, it is already clear that the UbE-WD40 interaction differs considerably from the WD40-DSGΦXS destruction motif interactions described until now.

Interaction of β-TrCP is the first step towards factual endocytosis of the GHR via a clathrin-mediated selection process. To involve the ubiquitination machinery in this process the GHR must be dimerised (Gent et al., 2002). Recent observations on β-TrCP show that it indeed contains a dimerisation domain that is important for its E3 function (Suzuki et al., 2000). Our *in vitro* binding experiments with GST-GHR tails reveal that monomeric GHR can bind β- rCP. It remains to be investigated whether the dimerisation of β-TrCP poses the necessity for dimerisation on the GHR.

Cargo selection via β-TrCP thus requires an active E3, followed by proteasomal activity, and interaction with the clathrin-mediated machinery ((Govers et al., 1999). Whether the full SCF β-TrCP E3 is in place to ubiquitinate factor X and enable GHR to connect to the propeller structure of the clathrin heavy chain remains open. Previously, we have identified several factors that bound specifically to the UbE motif (Schantl et al., 2003). The tetratricopeptide repeat-containing protein SGT might be a factor bridging the gap between TrCP and clathrin via the U-box ligase carboxyl-terminus of Hsc 70-interacting protein (CHIP) (Schantl et al., 2003; Timsit et al., 2005).

β-TrCP is a factor in the degradation of prolactin and interferon receptors (Kumar et al., 2003; Kumar et al., 2004; Li et al., 2004). Fuchs et al. identified β-TrCP to act upon and interact with these receptors via their DSGΦXS destruction motifs. They also show that SCF(β-TrCP2) E3 ubiquitinates both the interferon-α and prolactin receptors, dependent on an intact DSGΦXS motif. The assumption is that upon ligand binding (interferon-α and prolactin) this motif is phosphorylated by a yet unidentified kinase after which the SCF(β-TrCP2) E3 ubiquitinates certain lysines in the tail of the receptors. This leads to their destruction. It is clear that this scenario differs from how β-TrCP is involved in GHR endocytosis. Although the GHR contains a DSGRTS sequence, reminiscent to the DSGΦXS destruction motifs, mutation of the serine residues did not result in a shorter half-life nor in inhibition of the endocytosis of the GHR (unpublished). It is tempting to speculate that this sequence is involved in down-stream events originating from the action of y-secretase on the GHR tail (Cowan et al., 2005).

Ubiquitination by the SCF(β-TrCP) E3 of a factor generally results in its destruction by the proteasome. In most cases the decision to degrade the target is not in the SCF(β-TrCP) E3 action, but rather due to the activity of one or two kinases that phosphorylate the serine residues in the degradation motif. Our experiments with GST-GHR fusion proteins indicate that binding of β-TrCP to the UbE motif does not depend on a preceding action of another post-translation modification. Thus, very likely, the cytosolic concentration of β-TrCP determines its activity level to induce GHR endocytosis. This is reflected in the experiment in Fig. 1 that shows that decreased amounts of β-TrCP inhibit GH and GHR uptake. Thus, the cytosolic concentration of β-TrCP may function as a factor in GH-sensitivity of cells: at low concentrations the cells are more sensitive to GH than at high concentrations. This immediately raises the question what determines the cellular concentration of β-TrCP? Generally, β-TrCP is part of an E3 complex that is involved in degradation of key factors controlling cell growth, survival and transformation (Fuchs et al., 2004). As it is a short-lived protein itself, more studies are needed to understand the consequences of β-TrCP concentrations for cellular regulation, and the GHR activity in particular.

### Description of the figures

**Figure 1.** β-TrCP silencing inhibits ¹²⁵I-GH endocytosis. Hek293 cells expressing GHR were transfected with various siRNAs for three days. Cells were incubated in the presence of ¹²⁵I-GH on ice and uptake was measured for 10 min at 37°C. The cells were acid washed (to determine GH at the plasma membrane) and the acid-resistant radioactivity was taken as endocytosed GH. Internalized GH was expressed as a percentage of total radioactivity associated with the cells. Background values were determined by performing the experiment in the present of excess unlabeled GH and subtracted. Silencing of clathrin was used as a positive control. Silencing of other E3s (Traf2, Traf6), a ubiquitin conjugase (Ubc13), an endosomal sorting factor (TSG101), and Small glutamine-rich tetratricopeptide repeat-containing protein, a known GHR binding protein, did not affect GHR endocytosis.
**Figure 2.** Mutational analysis of the interaction between β-TrCP and the GHR. Hek293 cells transfected with GHR DNA constructs in which each of the UbE amino acids were mutated to alanine were lysed, the lysates were incubated with biotin-GH, and the GHRs were isolated using strepavidine-Agarose beads. The GH-GHR-containing beads were incubated with β-TrCP1, ³⁵S-labelled in an *in vitro* translation system. The beads were washed, and the amounts of radioactive β-TrCP2 bound to the beads were quantitated after separation on SDS- electrophoresis. Each lane contained approximately equal amount of GHR as quantified from the western blot using an Odyssey scanner. The values are expressed as percentages of binding to the wild-type receptor. Binding to the GHR truncated at amino acid residue 286 was subtracted before the calculation.

### References

Cowan, J. W., Wang, X., Guan, R., He, K., Jiang, J., Baumann, G., Black, R. A., Wolfe, M. S., and Frank, S. J. (2005). Growth Hormone Receptor Is a Target for Presenilin-dependent {gamma}-Secretase Cleavage. J Biol Chem 280, 19331-19342.

Fuchs, S. Y., Spiegelman, V. S., and Kumar, K. G. (2004). The many faces of beta-TrCP E3 ubiquitin ligases: reflections in the magic mirror of cancer. Oncogene 23, 2028-2036.

Galan, J. M., and Haguenauer-Tsapis, R. (1997). Ubiquitin Lys63 is involved in ubiquitination of a yeast plasma membrane protein. EMBO Journal 16, 5847-5854.

Gent, J., van Kerkhof, P., Roza, M., Bu, G., and Strous, G. J. (2002). Ligand-independent growth hormone receptor dimerization occurs in the endoplasmic reticulum and is required for ubiquitin system-dependent endocytosis. Proc Nat Acad Sci USA 99, 9858-9863.

Govers, R., ten Broeke, T., van Kerkhof, P., Schwartz, A. L., and Strous, G. J. (1999). Identification of a novel ubiquitin conjugation motif, required for ligand-induced internalization of the growth hormone receptor. EMBO J 18, 28-36.

Govers, R., van Kerkhof, P., Schwartz, A. L., and Strous, G. J. (1997). Linkage of the ubiquitin-conjugating system and the endocytic pathway in ligand-induced internalization of the growth hormone receptor. EMBO J 16, 4851-4858.

Hershko, A., Ciechanover, A., and Varshavsky, A. (2000). Basic Medical Research Award. The ubiquitin system. Nat Med 6, 1073-1081.

Hicke, L., and Dunn, R. (2003). Regulation of membrane protein transport by ubiquitin and ubiquitin-binding proteins. Annu Rev Cell Dev Biol 19, 141-172. Kanemori, Y., Uto, K., and Sagata, N. (2005). {beta}-TrCP recognizes a previously undescribed nonphosphorylated destruction motif in Cdc25A and Cdc25B phosphatases. PNAS 102, 6279-6284.

Kumar, K. G., Tang, W., Ravindranath, A. K., Clark, W. A., Croze, E., and Fuchs, S. Y. (2003). SCF(HOS) ubiquitin ligase mediates the ligand-induced down-regulation of the interferon-alpha receptor. Embo J 22, 5480-5490. Kumar, K. G. S., Krolewski, J. J., and Fuchs, S. Y. (2004). Phosphorylation and Specific Ubiquitin Acceptor Sites Are Required for Ubiquitination and Degradation of the IFNAR1 Subunit of Type I Interferon Receptor. J Biol Chem 279, 46614-46620.

Li, Y., Suresh Kumar, K. G., Tang, W., Spiegelman, V. S., and Fuchs, S. Y. (2004). Negative Regulation of Prolactin Receptor Stability and Signaling Mediated by SCF{beta}-TrCP E3 Ubiquitin Ligase. Mol Cell Biol 24, 4038-4048.

Plemper, R. K., and Wolf, D. H. (1999). Retrograde protein translocation: ERADication of secretory proteins in health and disease. Tr Biochem Sci 24, 266-270.

Roth, A. F., and Davis, N. G. (2000). Ubiquitination of the PEST-like endocytosis signal of the yeast a-factor receptor. Journal of Biological Chemistry 275, 8143-8153.

Schantl, J. A., Roza, M., de Jong, A. P., and Strous, G. J. (2003). Small glutamine-rich tetratricopeptide repeat-containing protein interacts with the ubiquitin-dependent endocytosis motif of the growth hormone receptor. Biochem J 373, 855-863.

Shenoy, S. K., McDonald, P. H., Kohout, T. A., and Lefkowitz, R. J. (2001). Regulation of Receptor Fate by Ubiquitination of Activated {beta}2-Adrenergic Receptor and {beta}-Arrestin. Science 294, 1307-1313.

Staub, O., Gautschi, I., Ishikawa, T., Breitschopf, K., Ciechanover, A., Schild, L., and Rotin, D. (1997). Regulation of stability and function of the epithelial Na+ channel (ENaC) by ubiquitination. EMBO J 16, 6325-6336.

Strous, G., and Gent, J. (2002). Dimerization, ubiquitylation and endocytosis go together in growth hormone receptor function. FEBS Lett 529, 102-109. Strous, G. J., and Govers, R. (1999). The ubiquitin-proteasome system and endocytosis. J Cell Science 112, 1417-1423.

Strous, G. J., van Kerkhof, P., Govers, R., Ciechanover, A., and Schwartz, A. L. (1996). The ubiquitin conjugation system is required for ligand-induced endocytosis and degradation of the growth hormone receptor. EMBO J 15, 3806-3812.

Suzuki, H., Chiba, T., Suzuki, T., Fujita, T., Ikenoue, T., Omata, M., Furuichi, K., Shikama, H., and Tanaka, K. (2000). Homodimer of two F-box proteins beta TrCP1 or beta TrCP2 binds to I kappa B alpha for signal-dependent ubiquitination. Journal of Biological Chemistry 275, 2877-2884.

Terrell, J., Shih, S., Dunn, R., and Hicke, L. (1998). A function for monoubiquitination in the internalization of a G protein-coupled receptor. Mol Cell 1, 193-202.

Timsit, Y. E., Miller, S. L., Mohney, R. P., and O'Bryan, J. P. (2005). The U-box ligase carboxyl-terminus of Hsc 70-interacting protein ubiquitylates Epsin. Biochem Biophys Res Commun 328, 550-559.

van Kerkhof, P., Smeets, M., and Strous, G. J. (2002). The Ubiquitin-Proteasome Pathway Regulates the Availability of the GH Receptor. Endocrinology 143, 1243-1252.

van Kerkhof, P., Vallon, E., and Strous, G. J. (2003). A method to increase the number of growth hormone receptors at the surface of cells. Mol Cell Endocrinol 201, 57-62.

Wu, G., Xu, G., Schulman, B. A., Jeffrey, P. D., Harper, J. W., and Pavletich, N. P. (2003). Structure of a beta-TrCP1-Skp1-beta-catenin complex: destruction motif binding and lysine specificity of the SCF(beta-TrCP1) ubiquitin ligase. Mol Cell 11, 1445-1456.

## Claims

1. A method for controlling the presence of a receptor at the surface of a cell comprising interfering with the interaction of an F-box protein with a ubiquitin/proteasome binding site of said receptor, wherein said ubiquitin/proteasome binding site comprises the amino acid sequence motif xDSWxEFIxxD or a sequence essentially corresponding thereto.

2. A method for increasing the presence of a receptor at the surface of a cell comprising providing to said cell an inhibitor that at least in part is capable of inhibiting the interaction of an F-box protein with a ubiquitin/proteasome binding site of said receptor, wherein said ubiquitin/proteasome binding site comprises the amino acid sequence motif xDSWxEFIxxD or a sequence essentially corresponding thereto.

3. A method according to claim 1 or 2, wherein said F-box protein is a β-transducing repeat-containing protein (β-TrCP).

4. A method according to any one of claims 1 to 3, wherein said receptor is a growth hormone receptor (GHR).

5. A method according to any one of claims 1 to 4, wherein said ubiquitin/proteasome binding site comprises the amino acid sequence motif DDSWVEFIELD or a sequence essentially corresponding thereto.

6. A method for controlling the presence of a growth hormone receptor (GHR) at the surface of a cell comprising interfering with the interaction of an F-box protein with a ubiquitin/proteasome binding site of said GHR.

7. A method for increasing the presence of a GHR at the surface of a cell comprising providing to said cell an inhibitor that at least in part is capable of inhibiting the interaction of an F-box protein with a ubiquitin/proteasome binding site of said GHR.

8. A method according to claim 6 or 7, wherein said F-box protein is a β-transducing repeat-containing protein (β-TrCP).

9. A method according to any one of claims 6 to 8, wherein said ubiquitin/proteasome binding site comprises the amino acid sequence motif xDSWxEFIxxD or a sequence essentially corresponding thereto.

10. A method according to any one of claims 6 to 9, wherein said ubiquitin/proteasome binding site comprises the amino acid sequence motif DDSWVEFIELD or a sequence essentially corresponding thereto.

11. A method for selecting an inhibitor capable of inhibiting the interaction of an F-box protein with a ubiquitin/proteasome binding site, comprising the steps of
- contacting, in the absence or presence of a possible inhibitor, said F-box protein or a functional part and/or equivalent thereof with a first labelled compound comprising at least a proteinaceous part comprising the amino acid sequence xDSWxEFIxxD or a sequence essentially corresponding thereto and
- determining the amount of said first labelled compound bound to said F-box protein in the presence or absence of said inhibitor and comparing the obtained amounts
- selecting the inhibitor that results in a decreased amount of said bound first labelled compound.

12. A method according to claim 11, further comprising immobilising said F-box protein or a functional part and/or equivalent thereof.

13. A method according to claim 11 or 12, further comprising
- contacting, in the absence or presence of the selected inhibitor, said F-box protein or a functional part and/or equivalent thereof with a second labelled compound comprising at least a proteinaceous part comprising the amino acid sequence DSGIHS or a sequence essentially corresponding thereto,
- determining the amount of said second labelled compound bound to said F-box protein in the presence or absence of said inhibitor and comparing the obtained amounts,
- selecting the inhibitor that does not or hardly not change the amount of said second labelled compound bound to said F-box protein.

14. A method according to any one of claims 11 to 13, further comprising testing the selected inhibitor in a cell-based assay.

15. A method according to any one of claims 11 to 14, wherein said F-box protein is β-TrCP.

16. A method according to claim 15, wherein said functional part of said F-box protein comprises the WD40-domain.

17. An inhibitor obtainable by a method according to any one of claims 11 to 16.

18. A pharmaceutical composition comprising an inhibitor according to claim 17.

19. Use of an inhibitor according to claim 17 for the manufacture of a medicament for treating diseases related to the availability of a cell surface receptor.

20. Use of an inhibitor according to claim 17 for the manufacture of a medicament for the treatment of (diseases which involve) cachexia.

21. Use according to claim 20, wherein said cachexia is a result of cancer, AIDS, renal insufficiency, burns or bed-ridden situations.


### Annex to the application documents - subsequently filed sequences listing

SEQUENCE LISTING

<110> Recharge B.V.

<120> Methods and means for controlling the endocytosis and subsequent
degradation of a cell surface protein

<130> P73706EP00

<140> EP 05076807.6
<141> 2005-08-02

<160> 12

<170> PatentIn version 3.3

<210> 1
<211> 11
<212> PRT
<213> Artificial

<220>
<223> Ubiquitin-dependent Endocytosis motif (UbE-motif)

<400> 1

Asp Asp Ser Trp Val Glu Phe Ile Glu Leu Asp
1               5                   10

<210> 2
<211> 6
<212> PRT
<213> Artificial

<220>
<223> Substrate binding motif

<220>
<221> MISC_FEATURE
<222> (4)..(5)
<223> "Xaa" on pos. 4 and 5 stands for any amino acid

<400> 2

Asp Ser Gly Xaa Xaa Ser
1               5

<210> 3
<211> 11
<212> PRT
<213> Artificial

<220>
<223> Ubiquitin/proteasome binding site

<220>
<221> MISC_FEATURE
<222> (1)..(10)
<223> "Xaa" on pos. 1, 5, 9 ,10 stands for any amino acid

<400> 3

Xaa Asp Ser Trp Xaa Glu Phe Ile Xaa Xaa Asp
1               5                   10

<210>  4
<211>  11
<212>  PRT
<213>  Artificial

<220>
<223>  Ubiquitin/proteasome binding site


<220>
<221>  MISC_FEATURE
<222>  (2)..(10)
<223>  "Xaa" on pos. 2, 5, 9 ,10 stands for any amino acid

<400>  4

Asp Xaa Ser Trp Xaa Glu Phe Ile Xaa Xaa Asp
1               5                   10


<210>  5
<211>  11
<212>  PRT
<213>  Artificial

<220>
<223>  Ubiquitin/proteasome binding site


<220>
<221>  MISC_FEATURE
<222>  (5)..(10)
<223>  "Xaa" on pos. 5, 9 ,10 stands for any amino acid

<400>  5

Asp Asp Ser Trp Xaa Glu Phe Ile Xaa Xaa Asp
1               5                   10


<210>  6
<211>  12
<212>  PRT
<213>  Artificial

<220>
<223>  Ubiquitin/proteasome binding site

<400>  6

Asp Asp Ser Trp Val Glu Phe Ile Glu Leu Asp Ile
1               5                   10


<210>  7
<211>  15
<212>  PRT
<213>  Artificial

<220>
<223>  Ubiquitin/proteasome binding site

<400>  7

Asn Asp Asp Ser Trp Val Glu Phe Ile Glu Leu Asp Ile Asp Asp
1               5                   10                  15

<210>  8
<211>  61
<212>  PRT
<213>  Artificial

<220>
<223>  Sequence of human 61 amino acid peptide

<400>  8

Lys Gln Gln Arg Ile Lys Met Leu Ile Leu Pro Pro Val Pro Val Pro
1               5                   10                  15

Lys Ile Lys Gly Ile Asp Pro Asp Leu Leu Lys Glu Gly Lys Leu Glu
            20                  25                  30

Glu Val Asn Thr Ile Leu Ala Ile His Asp Ser Tyr Lys Pro Glu Phe
        35                  40                  45

His Ser Asp Asp Ser Trp Val Glu Phe Ile Glu Leu Asp
    50                  55                  60

<210>  9
<211>  61
<212>  PRT
<213>  Artificial

<220>
<223>  Sequence of rabbit 61 amino acid peptide

<400>  9

Lys Gln Gln Arg Ile Lys Met Leu Ile Leu Pro Pro Val Pro Val Pro
1               5                   10                  15

Lys Ile Lys Gly Ile Asp Pro Asp Leu Leu Lys Glu Gly Lys Leu Glu
            20                  25                  30

Glu Val Asn Thr Ile Leu Ala Ile Gln Asp Ser Tyr Lys Pro Glu Phe
        35                  40                  45

Tyr Asn Asp Asp Ser Trp Val Glu Phe Ile Glu Leu Asp
    50                  55                  60

<210>  10
<211>  6
<212>  PRT
<213>  Artificial

<220>
<223>  Second labelled compound

<400>  10

Asp Ser Gly Ile His Ser
1               5

<210>  11
<211>  21
<212>  DNA
<213>  Artificial

<220>
<223>  Chemically synthesized siRNA duplex

<400>  11
guggaauuug uggaacauct t                                                21


<210>  12
<211>  21
<212>  DNA
<213>  Artificial

<220>
<223>  Chemically synthesized siRNA duplex

<400>  12
gauguuccac aaauuccact t                                                21